# EUROPEAN PATENT SPECIFICATION

(11) **EP 2 536 391 B1**
(45) Date of publication and mention of the grant of the patent: **27.12.2017**
(21) Application number: 11705784.4
(22) Date of filing: 17.02.2011
(51) Int. Cl.: A61K 8/92, A61Q 9/02, A61Q 19/00, A61K 8/90, A61K 8/37, A61K 8/31

(54) **HAIR REMOVAL DEVICE COMPRISING AN ERODABLE COMPOSITION**
HAARENTFERNUNGSVORRICHTUNG MIT ERODIERBARER ZUSAMMENSETZUNG
DISPOSITIF D'ÉPILATION COMPRENANT UNE COMPOSITION ÉRODABLE

(30) Priority: 18.02.2010 US 305687 P
(43) Date of publication of application: 26.12.2012
(73) Proprietor: The Gillette Company LLC, Boston, MA 02127 (US)
(72) Inventor: STEPHENS, Alison, Fiona, Maidenhead Berkshire SL6 9LA (GB); BROOKS, Alan, Woking Surrey GU21 8UQ (GB); KWIECIEN, Michael, Joseph, Scituate, Massachusetts 02066 (US)
(74) Representative: Kohol, Sonia
(86) International application number: PCT/US2011/025151
(87) International publication number: WO 2011/103235

(56) References cited:
- WO-A1-94/12190
- WO-A1-97/02116
- WO-A1-99/28429
- WO-A1-2007/056509
- WO-A1-2008/045451
- US-A1- 2006 272 155

## Description

### FIELD OF THE INVENTION

The present invention concerns the provision of a hair removal device comprising an erodable, solid moisturizing composition.

### BACKGROUND OF THE INVENTION

Hair removal devices incorporating a chemical composition are known and shall be referred to herein as devices comprising an "onboard" composition. Reference can be made to WO 07/056509 which teaches the inclusion of an onboard soap composition in a wet shaving razor. It is also known to provide a wet shaving razor incorporating an onboard skin-engaging composition comprising large quantities of hydrophilic polymers, such as polyethylene oxide, to lubricate the skin. Reference is made, by way of example, to WO 97/02116 and WO 97/02117.

The patent applications referred to above relate to the provision of various advantages, such as additional lathering and soap-related benefits, or improved lubrication in the case of polyethylene oxide. It would alternatively or additionally be advantageous to be able to provide a skin moisturizing benefit via an onboard chemistry, especially to male users who may be less motivated to use skin moisturizers than females. The provision of a moisturizing benefit from an onboard chemistry may, however, have a number of difficulties associated with it.

Skin moisturization may be achieved in several different ways, but one important formulational route to achieving skin moisturization is to include materials which bind water, such as polyols. However, polyols derive their water-binding abilities in part from their significant hydrophilicity, which may render them unsuited for use in any context involving water, such as is the case during wet shaving - they may be washed away during the initial stages of a shave. An alternative approach might be to use occlusive, hydrophobic materials which cover the skin and therefore act to retain water already present in the skin. These materials are occlusive hydrophobic emollients which are less likely to be washed away during use in a highly aqueous environment. WO 06/108522, discloses the use of small amounts of hydrophobic emollients in an onboard chemistry. However, formulations comprising significant proportions of hydrophobic emollients generally have mechanical and/or rheological properties which render them unsuitable for use as an onboard chemistry. For example, many hydrophobic emollients are liquids or low viscosity gels which flow easily and/or whose integrity may become substantially impaired by contact with hair, especially stiff male beard hair. One possible result, is that the emollient formulation may be mostly or entirely delivered at the start of the first use, over-delivering for that first use and leaving little or nothing for future uses of the hair removal device. Other hydrophobic emollients, such as some waxes, may not confer sufficient flexibility to an emollient composition. A further perceived problem associated with compositions comprising significant proportions of hydrophobic emollients is that they may increase drag across the skin, due to their affinity with the hydrophobic skin surface. An increase in friction is perceived as being a problem, because it is believed to result in a less pleasant hair removal experience.

### SUMMARY OF THE INVENTION

According to a first aspect of the invention, a hair removal device is provided, comprising an erodable, solid moisturizing composition, the erodable, solid moisturizing composition comprising:
a. A structuring polymer;
b. At least 50% moisturizing hydrophobic phase by weight of the erodable, solid moisturizing composition, wherein at least 40% by weight of the moisturizing hydrophobic phase comprises materials which are liquid at 25°C as defined in claim 1.

According to a second aspect of the invention, the use of a hair removal device according to the first aspect to remove hair and moisturize the skin is provided.

### DETAILED DESCRIPTION OF THE INVENTION

The "moisturizing hydrophobic phase", as herein defined, does not comprise and is separate from the structuring polymer.

As used herein, the term "solid" when used in relation to the erodable, solid moisturizing composition refers to compositions which are solid at 25°C.

As used herein, the term "water-insoluble" when used in relation to the structuring polymer, means "very slightly soluble", according to the United States' Pharmacopeia (USP) definition in 31/NF 26 Vol. 2 General Notices, Page Xvii., or less than "very slightly soluble", which, using the USP definition, means that more than 1000 parts of solvent (water, in this case) are needed to dissolve 1 part of solute (the structuring polymer, in this case) at Standard Temperature and Pressure.

As used herein, the term "soluble in" when describing the ability of the structuring polymer to dissolve in the moisturizing hydrophobic phase means "soluble", according to the United States' Pharmacopeia definition in 31/NF 26 Vol. 2 General Notices, Page Xvii., or less than "soluble", which, using the USP definition, means that less than 30 parts of solvent (the moisturizing hydrophobic phase, in this case) are needed to dissolve 1 part of solute (the structuring polymer, in this case) at the melting point of the structuring polymer.

The hair removal device according to the invention may be any hair removal device, such as, but not limited to, a razor, an epilator or a hair removal device comprising a light source to degrade or destroy the hair and/or hair root. If the hair removal device comprises a light source, then the light source may generate coherent (laser) light or incoherent light and may be adapted to generate light continuously or in a discontinuous (pulsed) fashion.

Preferably, the hair removal device is a razor. In the case of a razor, then the solid moisturizing composition may advantageously be provided on the razor cartridge, is preferably configured as a strip located before and/or after the blade(s) in the direction of cutting and may even be configured as a ring entirely surrounding the blades. WO 97/02116, referred to above, illustrates locations in which such a strip may be placed. Advantageously, the solid moisturizing composition is configured as a strip disposed after the cutting blade(s) in the cutting direction and, in this case, a lubricating strip comprising polyalkylene glycol is preferably additionally disposed before the cutting blades in the cutting direction.

The present applicants have established that a hair removal device comprising an erodable, solid moisturizing composition rich in hydrophobic materials, but also comprising a structuring polymer to bestow appropriate wear properties to the moisturizing hydrophobic phase, may deposit an effective amount of hydrophobic materials onto the skin during a hair removal process and that it may do this during a plurality of successive uses of a hair removal device. Surprisingly, the present applicants have also established that the moisturizing hydrophobic phase, while increasing drag, is actually appreciated by consumers, since it may assist in tensioning the skin, which is known to assist in the hair removal process.

Advantageously, the erodable, solid moisturizing composition comprised within a hair removal device according to the invention has a Chatillon Hardness at 25°C of 0.50 - 3.25kg, preferably 0.75 - 3.00kg, more preferably 1.00 - 2.50kg, measured according to the protocol provided hereinbelow. Within these ranges, beneficial rates of wear may be achieved.

The structuring polymer comprised within the erodable, solid moisturizing composition may be any polymer which bestows appropriate wear properties to the erodable, solid moisturizing composition and is preferably a polymer which may bestow a Chatillon Hardness in the above-defined ranges to the erodable, solid moisturizing composition.

According to one advantageous embodiment, the structuring polymer is water-insoluble to assist miscibility with or solubility in the moisturizing hydrophobic phase (at the melting point of the water-insoluble polymer). If the structuring polymer is miscible with or soluble in the moisturizing hydrophobic phase, then this may ensure a homogenous distribution of polymer throughout the moisturizing hydrophobic phase and thus more even wear properties. In addition, a water-insoluble structuring polymer may improve the durability of the polymer (and therefore also the erodable solid moisturizing composition) versus more hydrophilic polymers which may solubilise and wash away during hair removal processes that employ water, such as wet shaving.

According to the invention, the erodable, solid moisturizing composition comprises from 2% to 50%, preferably from 3% to 40%, more preferably 4% to 12% of structuring polymer by weight of the erodable, solid moisturizing composition.

The structuring polymer comprises a block copolymer. The block copolymer comprises a di-block copolymer, a tri-block copolymer, a multi-block copolymer, a radial block copolymer, a random block copolymer, or a mixture of these polymers. More advantageously still, the block copolymer comprises a tri-block copolymer.

In the case in which the block copolymer comprises a tri-block copolymer, then the tri-block copolymer preferably comprises a linear ABA tri-block polymer. Without wishing to be bound by theory, applicants believe that the A blocks aggregate creating domains, within which the moisturizing hydrophobic phase may accumulate, connected together by the B-blocks. This structure may provide an appropriate hardness to bestow the requisite wear properties to the erodable, solid moisturizing composition, while also being flexible enough to be processed and not to crack or break during processing and/or use.

Advantageously, the linear ABA block copolymer comprises styrene-butadiene-styrene (SBS) block copolymer, styrene-isoprene-styrene (SIS) block copolymer, styrene-ethylenebutylene-styrene (S-EB-S) block copolymer, or mixtures thereof. More advantageously, the linear ABA block copolymer preferably comprises styrene-ethylenebutylene-styrene (S-EB-S) block copolymer. More advantageously still, the weight ratio of styrene to butadiene in the S-EB-S is in the range 20:80 to 40:60 and preferably around 30:70.

Particularly useful commercially available ABA block copolymers include Versagel™ materials available from Penreco and the Kraton™ G series, especially G-6150, G-1651, G-1652 and 1654.

As discussed above, the structuring polymer comprised within the erodable, sold moisturizing composition may comprise a random block copolymer. An example of a suitable random block copolymer is ethylene vinyl acetate (EVA) which is a copolymer of ethylene and vinyl acetate. Advantageously, the amount of ethylene comprised within the EVA polymer is from 65-90%, preferably from 70-85% by weight of the EVA to give beneficial wear properties. A commercially available range of EVA is called Elvax™, which is commercialised by DuPont.

According to the invention, the erodable, solid moisturizing composition comprises at least 50% moisturizing hydrophobic phase by weight of the erodable, solid moisturizing composition. Preferably, the erodable, solid moisturizing composition comprises from 60% to 95% and more preferably from 70% to 90% moisturizing hydrophobic phase by weight of the erodable, solid moisturising composition.

The moisturizing hydrophobic phase may comprise emollient which is liquid, semi-solid and/or solid at room temperature, provided that at least 40%, preferably at least 60%, more preferably at least 70% by weight of the moisturizing hydrophobic phase comprises materials which are liquid at 25°C. Applicants have observed that high melting point hydrophobic materials are not delivered as evenly and consistently as low melting point ones. Without wishing to be bound by theory, applicants believe that this has to do with the reduced tendency to spread of higher melting point hydrophobic materials. In addition, applicants have observed that a very high proportion of high melting point hydrophobic materials may give more uneven erosion of the solid moisturizing composition. Suitable materials to make up the liquid portion of the moisturizing hydrophobic phase are listed below. Preferred liquids comprise mineral oil, fractionated coconut oil, isopropyl isostearate, isopropyl myristate and mixtures thereof. Advantageously, no more than 80% by weight of the moisturizing hydrophobic phase comprises materials which are liquid at 25°C.

Advantageously, if solids or semi-solids are present, then the moisturizing hydrophobic phase comprises less than 20% and preferably less than 5% by weight of the solid moisturizing composition of materials, and more preferably no materials at all, having a melting point of more than 100°C. This is because excessive quantities of such materials may render the composition inflexible and therefore liable to crack during manufacture and/or use.

The moisturizing hydrophobic phase may comprise one or more hydrocarbons, fatty acids, fatty alcohols, esters, triglycerides, fats, butters, waxes, lipophilic skin active agents or mixtures thereof.

Liquid, semi-solid, or solid hydrocarbon emollients which may be comprised within the erodable, sold moisturizing composition include straight chain, branched chain, saturated and unsaturated hydrocarbons and mixtures thereof and they may comprise natural or synthetic hydrocarbon emollients and mixtures thereof. Preferred natural hydrocarbon emollients include petrolatum, mineral oil and mixtures thereof. Preferred synthetic hydrocarbon emollients include branched chain hydrocarbons, such as isohexadecane (such as Arlamol HD™ from Croda) and Polydecene (such as Puresyn 2™ from Exxon Mobil).

Liquid, semi-solid, or solid fatty alcohol or fatty acid emollients which may be comprised within the erodable, sold moisturizing composition include saturated and unsaturated higher alcohols, especially C₁₂ - C₃₀ fatty alcohols and fatty acids, especially lauric, myristic, palmitic, stearic , arachidic or behenic.

Liquid, semi-solid, or solid ester emollients which may be comprised within the erodable, sold moisturizing composition include esters of a C₁₂ - C₃₀ alcohol and mixtures thereof, especially isopropyl myristate, isopropyl isostearate and mixtures thereof.

Liquid, semi-solid, or solid triglyceride emollients which may be comprised within the erodable, sold moisturizing composition include synthetic or natural triglycerides, especially natural triglycerides derived from sunflower, avocado, olive, castor, coconut, cocoa and mixtures thereof. More preferred are coconut-derived triglycerides, such as the commercially available materials Myritol™ 312 and 318 (Cognis), Estasan™ (Croda) and Miglyol™ (Sasol).

Liquid, semi-solid, or solid fat and butter emollients which may be comprised within the erodable, sold moisturizing composition include coconut butter, shea butter and mixtures thereof.

Liquid, semi-solid, or solid wax emollients which may be comprised within the inventive composition include paraffin wax, microcrystalline wax, candellila, ozokerite and mixtures thereof. Preferably the emollient comprises paraffin wax. Advantageously, moisturizing hydrophobic phase comprises some wax because waxes may bestow further improved hardness and erodability to the solid moisturising composition, although, as discussed above, the presence of too much wax may render the composition inflexible and therefore liable to crack during manufacture and/or use. Preferably, the solid moisturising composition comprises from 2% to 20% and more preferably from 3% to 15% wax by weight of the solid moisturising composition.

Liquid, semi-solid, or solid lipophilic skin active agent emollients which may be comprised within the inventive composition include oil soluble vitamins, such as vitamin E derivatives, including vitamin E acetate and tocopherol nicotinate; oil-soluble vitamin A derivatives, such as retinyl palmitate; lanolin; ceramides; sterols and sterol esters; salicylic acid; camphor; eucalyptol; essential oils and mixtures thereof.

The erodable, sold moisturizing composition may comprise one or more additional components which bestow a suitable melt viscosity to the composition, such as oil phase gellants, to facilitate improved processing, provided that the additional component(s) do not significantly reduce the hardness or erodability of the erodable, sold moisturizing composition. Examples of such components are trihydroxystearin, which is commercially available as Thixcin R™ (manufactured by Elementis Specialities), ethylene vinyl acetate (EVA) and mixtures thereof.

A erodable, sold moisturizing composition comprised within the hair removal device of the invention may be manufactured by heating the elements of the moisturizing hydrophobic phase to a suitable temperature to melt them, typically approximately 130°C, after which the structuring polymer is added and mixed well until the structuring polymer has dissolved. The mixture is then cooled, typically to approximately 90°C, after which any additional ingredients may be added. In a final step, the mixture is poured into suitable containers or moulds and allowed to cool to room temperature.

Once the mixture has set, it may be affixed to a hair removal device in any appropriate fashion. One such approach would involve mechanically fitting the composition onto the hair removal device. Alternatively, the composition may be directly or indirectly adhered to the hair removal device by means of an adhesive composition. One method of indirect adherence involves casting the solid moisturizing composition onto a sheet of an appropriate substrate, such as an acetate sheet, which sheet is then adhered to the hair removal device, for example mechanically or via an adhesive.

### Chatillon Hardness test

Equipment: Chatillon TCD 200 equipped with a digital force gauge

### Sample preparation

1. Fully melt and cast lipid into 60ml weigh boat (70mm X 70mm X 24mm)
2. Store lipid at 25°C overnight to equibrilate
3. Carefully remove lipid from weigh boat prior to hardness testing

### Machine Preparation

### A)

1. Prepare Chatillon TCD 200 and digital force gauge according to manufacturers instructions.
2. Set the ramp speed to 47 mm / min

### B) Measuring the hardness value at 25°C:

1. The pointed geometry should be attached to the shaft of ramp for this test method.
2. Place the lipid sample as prepared above and on its side onto the metal base plate directly below the centre of the shaft of the ramp. The mid-point of the lipid should be in line with the centre of the shaft of the ramp.
3. With the lipid in place below the flat plate the speed set at 47 mm/min and the digital force gauge set at "C Peak" as above, depress the "Down" button on the Chatillon TCD200.
4. Stop the Chatillon TCD200 just as the probe touches the surface of the lipid and set the distance counter to zero.
5. Reset the force gauge so that it reads zero
6. Depress the "Down" button on the Chatillon TCD200 until the distance counter reads 13mm, record C Peak reading.

### Examples

The following examples disclose solid moisturizing compositions, which were incorporated into razors as a strip disposed after the blade in the cutting direction. In use, they were observed to remove hair and deposit the solid moisturizing composition onto the skin from which hair had been removed.

### Example 1

| **Trade Name** | **INCI Name** | **% w/w** |
|---|---|---|
| White soft paraffin | Petrolatum | 44.0 |
| Mineral oil | Paraffinum Liquidum | 44.0 |
| Kraton G1650E | Hydrogenated Styrene/Butadiene copolymer | 5.0 |
| Thixcin R | Trihydroxystearin | 2.0 |
| Paraffin Wax SP206 | Paraffin | 5.0 |

The composition of Example 1 is manufactured by heating the hydrocarbons and waxes to 130°C, then adding the linear ABA tri-block polymer (Kraton G1650E) and mixing well until polymer has fully dissolved. The mixture is then cooled to 90°C and the Thixcin added, after which the mixture is poured into a mould and allowed to cool to room temperature.

The Chatillon Hardness of the formulation of Example 1 is 1.7.

### Example 2

| **Trade Name** | **INCI Name** | **% w/w** |
|---|---|---|
| Fractionated coconut oil | Caprylic/Capric Triglyceride | 85.0 |
| Paraffin Wax SP206 | Paraffin | 7.5 |
| Kraton GRP6935 | Hydrogenated Styrene/Butadiene copolymer | 5.0 |
| Synthetic Beeswax | Arachidyl Behenate | 2.5 |

The composition of Example 2 is manufactured by heating the hydrocarbons and waxes to 130°C, then adding the linear ABA tri-block polymer (Kraton GRP6935) and mixing well until polymer has fully dissolved. The mixture is then cooled to 90°C, after which the mixture is poured into a mould and allowed to cool to room temperature.
The Chatillon Hardness of the formulation of Example 2 is 0.6.

### Example 3

| **Trade Name** | **INCI Name** | **% w/w** |
|---|---|---|
| White soft paraffin | Petrolatum | 40.0 |
| Mineral oil | Paraffinum Liquidum | 40.0 |
| Kraton G1650E | Hydrogenated Styrene/Butadiene copolymer | 10.0 |
| Paraffin Wax SP206 | Paraffin | 10.0 |

The composition of Example 3 is manufactured by heating the hydrocarbons and wax to 130°C, then adding the linear ABA tri-block polymer (Kraton G1650E) and mixing well until polymer has fully dissolved. The mixture is then cooled to 90°C, after which the mixture is poured into a mould and allowed to cool to room temperature.

### Example 4

| **Trade Name** | **INCI Name** | **% w/w** |
|---|---|---|
| White soft paraffin | Petrolatum | 39.0 |
| Mineral oil | Paraffinum Liquidum | 39.0 |
| Paraffin Wax SP206 | Paraffin | 10.0 |
| Elvax 205W | Ethylene/Vinyl Acetate (EVA) copolymer | 10.0 |
| Thixcin R | Trihydroxystearin | 2.0 |

The composition of Example 4 is manufactured by heating the hydrocarbons and wax to 130°C, then adding the linear ABA tri-block polymer (Kraton G1650E) and the ethyle/vinyl acetate copolymer (Elvax 205W) and mixing well until polymer has fully dissolved. The mixture is then cooled to 90°C and the Thixcin added, after which the mixture is poured into a mould and allowed to cool to room temperature.

The Chatillon Hardness of the formulation of Example 4 is 1.25.

The dimensions and values disclosed herein are not to be understood as being strictly limited to the exact numerical values recited. Instead, unless otherwise specified, each such dimension is intended to mean both the recited value and a functionally equivalent range surrounding that value. For example, a dimension disclosed as "40 mm" is intended to mean "about 40 mm".

## Claims

1. A hair removal device comprising an erodable, solid moisturizing composition, the erodable, solid moisturizing composition comprising:
a. from 2% to 50% of a water insoluble structuring polymer, by weight of the erodable, solid moisturizing composition, wherein the structuring polymer comprises a block copolymer, wherein the block copolymer comprises a di-block copolymer, a tri-block copolymer, a multi-block copolymer, a radial block copolymer, a random block copolymer, or a mixtures of these polymers,
b. At least 50% of a moisturizing hydrophobic phase, by weight of the erodable, solid moisturizing composition, wherein at least 40%, by weight of the moisturizing hydrophobic phase comprises materials which are liquid at 25°C, wherein said liquid materials comprise mineral oil, fractionated coconut oil, isopropyl isostearate, isopropyl myristate or mixtures thereof.

2. The hair removal device of claim 1, wherein the erodable, solid moisturizing composition has a Chatillon Hardness of 0.50kg to 3.50kg, preferably 0.75kg to 3.00kg, more preferably 1.00kg to 2.50 kg (as described herein).

3. The hair removal device of either preceding claim, wherein the erodable, solid moisturizing composition comprises from 3% to 40%, more preferably 4% to 12% structuring polymer by weight of the erodable, solid moisturizing composition.

4. The hair removal device according to claim 1, wherein said moisturising hydrophobic phase composition comprises at least 60%, preferably at least 70% by weight of the moisturising hydrophobic phase of materials which are liquid at 25°C.

5. The hair removal device of claim 1, wherein the block copolymer comprises a linear ABA tri-block copolymer.

6. The hair removal device of claim 1, wherein the linear ABA block copolymer comprises styrene-butadiene-styrene (SBS) block copolymer, styrene-isoprene-styrene (SIS) block copolymer, styrene-ethylenebutylene-styrene (S-EB-S) block copolymer, or mixtures thereof.

7. The hair removal device of any preceding claim, wherein the erodable, solid moisturizing composition comprises from 60% to 95%, preferably from 70% to 90% moisturizing hydrophobic phase by weight of the solid moisturising composition.

8. The hair removal device of any preceding claim, wherein the moisturizing hydrophobic phase comprises petrolatum, esters, triglycerides, waxes or mixtures thereof.

9. The hair removal device of any preceding claim, wherein the moisturizing hydrophobic phase comprises wax and preferably the solid moisturising composition comprises from 2% to 20% and more preferably from 3% to 15% wax by weight of the solid moisturising composition.

10. The hair removal device of any preceding claim, configured as a razor.

11. The razor according to claim 10, wherein the solid moisturizing composition is configured as a strip disposed on one or both sides of the cutting blade(s).

12. Use of a hair removal device according to any preceding claim to remove hair and moisturize the skin.

## Patentansprüche

1. Haarentfernungsvorrichtung, umfassend eine erodierbare, feste feuchtigkeitsspendende Zusammensetzung, wobei die erodierbare feste feuchtigkeitsspendende Zusammensetzung Folgendes umfasst:
a. von 2 % bis 50 % eines wasserunlöslichen Strukturierungspolymers, pro Gewichtseinheit der erodierbaren festen feuchtigkeitsspendenden Zusammensetzung, wobei das Strukturierungspolymer ein Block-Copolymer umfasst, wobei das Block-Copolymer ein Di-Block-Copolymer, ein Tri-Block-Copolymer, ein Multi-Block-Copolymer, ein Radial-Block-Copolymer, ein Random-Block-Copolymer oder Mischungen dieser Polymere umfasst,
b. mindestens 50 % einer feuchtigkeitsspendenden hydrophoben Phase, pro Gewichtseinheit der erodierbaren festen feuchtigkeitsspendenden Zusammensetzung, wobei mindestens 40 Gew.-% der feuchtigkeitsspendende hydrophoben Phase Materialien umfasst, die bei 25°C flüssig sind, wobei die flüssigen Materialien Mineralöl, fraktioniertes Kokosöl, Isopropyl-Isostearat, Isopropyl-Myristat oder Mischungen daraus umfassen.

2. Haarentfernungsvorrichtung nach Anspruch 1, wobei die erodierbare, feste feuchtigkeitsspendende Zusammensetzung eine Chatillon-Härte von 4,90 N bis 34,32 N (von 0,50 kg bis 3,50 kg) aufweist, vorzugsweise 7,35 N bis 29,42 N (0,75 kg bis 3,00 kg), mehr bevorzugt 9,81 N bis 24,52 N (1,00 kg bis 2,50 kg) (entsprechend der Beschreibung hierin).

3. Haarentfernungsvorrichtung nach einem der vorstehenden Ansprüche, wobei die erodierbare, feste feuchtigkeitsspendende Zusammensetzung von 3 Gew.-% bis 40 Gew.-%, mehr bevorzugt von 4 Gew.-% bis 12 Gew.-%, Strukturierungspolymer der erodierbaren, festen feuchtigkeitsspendenden Zusammensetzung umfasst.

4. Haarentfernungseinrichtung nach Anspruch 1, wobei die feuchtigkeitsspendende hydrophobe Phase mindestens 60 Gew.-%, vorzugsweise mindestens 70 Gew.-% der feuchtigkeitsspendenden hydrophoben Phase der Substanzen umfasst, die bei 25 °C flüssig sind.

5. Haarentfernungseinrichtung nach Anspruch 1, wobei das Block-Copolymer ein lineares ABA-Tri-Block-Copolymer umfasst.

6. Haarentfernungseinrichtung nach Anspruch 1, wobei das lineare ABA-Block-Copolymer ein Styrol-Butadien-Styrol-(SBS)-Block-Copolymer, ein Styrol-Isopren-Styrol-(SIS)-Block-Copolymer, ein Styrol-Ethylenbutylen-Styrol-(S-EB-S)-Block-Copolymer oder Mischungen daraus umfasst.

7. Haarentfernungseinrichtung nach einem der vorstehenden Ansprüche, wobei die erodierbare, feste feuchtigkeitsspendende Zusammensetzung von 60 Gew.-% bis 95 Gew.-%, vorzugsweise von 70 Gew.-% bis 90 Gew.-%, feuchtigkeitsspendende hydrophobe Phasen der festen feuchtigkeitsspendenden Zusammensetzung umfasst.

8. Haarentfernungseinrichtung nach einem der vorstehenden Ansprüche, wobei die feuchtigkeitsspendende hydrophobe Phase Petrolatum, Ester, Triglyceride, Wachse oder Mischungen daraus umfasst.

9. Haarentfernungseinrichtung nach einem der vorstehenden Ansprüche, wobei die feuchtigkeitsspendende hydrophobe Phase Wachs umfasst und die feste feuchtigkeitsspendende Zusammensetzung von 2 Gew.-% bis 20 Gew.-% und mehr bevorzugt von 3 Gew.-% bis 15 Gew.-% der festen feuchtigkeitsspendenden Zusammensetzung umfasst.

10. Haarentfernungsvorrichtung nach einem der vorstehenden Ansprüche, die als Rasierer ausgeführt ist.

11. Rasierer nach Anspruch 10, wobei die feste feuchtigkeitsspendende Zusammensetzung als ein Streifen ausgeführt ist, der auf einer Seite oder auf beiden Seiten des/der Schneidmesser(s) angeordnet ist.

12. Verwendung einer Haarentfernungsvorrichtung nach einem der vorstehenden Ansprüche, zum Entfernen von Haaren und um der Haut Feuchtigkeit zu spenden.

## Revendications

1. Dispositif d'épilation comprenant une composition hydratante solide érodable, la composition hydratante solide érodable comprenant :
a. de 2 % à 50 % d'un polymère structurant insoluble dans l'eau, en poids de la composition hydratante solide érodable, dans lequel le polymère structurant comprend un copolymère séquencé, dans lequel le copolymère séquencé comprend un copolymère diséquencé, un copolymère triséquencé, un copolymère multiséquencé, un copolymère séquencé radial, un copolymère séquencé statistique, ou des mélanges de ces polymères,
b. au moins 50 % d'une phase hydrophobe hydratante, en poids de la composition hydratante solide érodable, dans lequel au moins 40 % en poids de la phase hydrophobe hydratante comprend des matériaux qui sont liquides à 25 °C, dans lequel lesdits matériaux liquides comprennent une huile minérale, de l'huile de coco fractionnée, de l'isostéarate d'isopropyle, du myristate d'isopropyle ou des mélanges de ceux-ci.

2. Dispositif d'épilation selon la revendication 1, dans lequel la composition hydratante solide érodable a une dureté Chatillon de 4,90 N à 34,32 N (de 0,50 kg à 3,50 kg), de préférence 7,35 N à 29,42 N (0,75 kg à 3,00 kg), plus préférablement 9,81 N à 24,52 N (1,00 kg à 2,50 kg) (telle que décrite ici).

3. Dispositif d'épilation selon l'une ou l'autre revendication précédente, dans lequel la composition hydratante solide érodable comprend de 3 % à 40 %, plus préférablement 4 % à 12 % de polymère structurant en poids de la composition hydratante solide érodable.

4. Dispositif d'épilation selon la revendication 1, dans lequel ladite phase hydrophobe hydratante comprend au moins 60 %, de préférence au moins 70 % en poids de la phase hydrophobe hydratante de matériaux qui sont liquides à 25 °C.

5. Dispositif d'épilation selon la revendication 1, dans lequel le copolymère séquencé comprend un copolymère triséquencé ABA linéaire.

6. Dispositif d'épilation selon la revendication 1, dans lequel le copolymère séquencé ABA linéaire comprend un copolymère séquencé styrène-butadiène-styrène (SBS), un copolymère séquencé styrène-isoprène-styrène (SIS), un copolymère séquencé styrène-éthylènebutylène-styrène (S-EB-S), ou leurs mélanges.

7. Dispositif d'épilation selon une quelconque revendication précédente, dans lequel la composition hydratante solide érodable comprend de 60 % à 95 %, de préférence de 70 % à 90 % de phase hydrophobe hydratante en poids de la composition hydratante solide.

8. Dispositif d'épilation selon une quelconque revendication précédente, dans lequel la phase hydrophobe hydratante comprend de la vaseline, des esters, des triglycérides, des cires ou leurs mélanges.

9. Dispositif d'épilation selon une quelconque revendication précédente, dans lequel la phase hydrophobe hydratante comprend de la cire et, de préférence, la composition hydratante solide comprend de 2 % à 20 % et plus préférablement de 3 % à 15 % de cire en poids de la composition hydratante solide.

10. Dispositif d'épilation selon l'une quelconque des revendications précédentes, configuré sous forme de rasoir.

11. Rasoir selon la revendication 10, dans lequel la composition hydratante solide est conçue sous forme de bande disposée sur l'un et/ou l'autre des côtés de la ou des lame(s) de coupe.

12. Utilisation d'un dispositif d'épilation selon l'une quelconque des revendications précédentes pour éliminer les poils et hydrater la peau.
